Europäisches Patentamt

European Patent Office

Office européen des brevets

(11) Publication number: **0 336 405 B1**

(12) **EUROPEAN PATENT SPECIFICATION**

(45) Date of publication of patent specification: **13.01.93** (51) Int. Cl.5: **A61K 47/48, C12P 21/08**

(21) Application number: **89106024.6**

(22) Date of filing: **06.04.89**

(54) Anti-human cancer protein complexes, their production and use.

(30) Priority: **08.04.88 JP 87845/88**
**21.02.89 JP 41323/89**

(43) Date of publication of application:
**11.10.89 Bulletin 89/41**

(45) Publication of the grant of the patent:
**13.01.93 Bulletin 93/02**

(84) Designated Contracting States:
**AT BE CH DE ES FR GB GR IT LI LU NL SE**

(56) References cited:
**EP-A- 0 305 960**
**WO-A-83/03679**
**WO-A-87/01941**
**WO-A-88/00703**
**US-A- 4 545 985**

(73) Proprietor: **Takeda Chemical Industries, Ltd.**
**1-1, Doshomachi 4-chome**
**Chuo-ku, OSAKA(JP)**

Proprietor: **Director-General of the Agency of**
**Industrial Science and Technology**
**Ministry of International Trade & Industry 3-1**
**Kasumigaseki 1-chome**
**Chiyoda-ku Tokyo 100(JP)**

(72) Inventor: **Iwasa, Susumu**
**21-2, Ohsumigaoka 1-chome Tanabe-cho**
**Tsuzuki-gun Kyoto 610-03(JP)**
Inventor: **Kuriyama, Masato**
**38-512, 1 Uchin-daicho 4-chome**
**Miyakojima-ku Osaka 534(JP)**
Inventor: **Okazaki, Kimitake**
**302, 14-13, Takezono 2-chome Tsukuba**
**Ibaraki 305(JP)**
Inventor: **Toyoda, Yukio**
**8-8, Mizudocho 1-chome**
**Amagasaki Hyogo 661(JP)**

(74) Representative: **von Kreisler, Alek,**
**Dipl.-Chem. et al**
**Patentanwälte Von Kreisler-Selting-Werner,**
**Deichmannhaus am Hauptbahnhof**
**W-5000 Köln 1(DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**Description**

BACKGROUND OF THE INVENTION

The present invention relates to a new protein complex which functions well as a therapeutic drug for cancer, specifically to an anti-human cancer protein complex which comprises (a) a ligand which is capable of binding to human cancer cells, (b) a ligand which is capable of binding to P*seudomonas aeruginosa* exotoxin A (hereinafter also abbreviated PEA) and (c) P*seudomonas aeruginosa* exotoxin A(PEA) coupled to the ligand (b), and a method of production of said protein complex.

Cancer treatment has long been studied in various investigations. A number of compounds have been proposed for use as chemotherapeutics with the aim of selective destruction of cancer cells. These attempts have resulted in successes to some extent; however, nonspecific toxicity of such compounds to normal highly proliferative cells, e.g. blood stem cells, has often been observed. Applicability has therefore been limited; particularly, these compounds have been unsatisfactory for use in eliminating cancer cells in terminal cases.

With the aim of mitigating such nonspecific toxicity of anticancer agents to normal cells, namely side effects, attempts have been made to use antibodies showing directivity for cancer cells as anticancer agent carrier molecules to fix the anticancer agent on the target cells. The anticancer agents used for this purpose are cytotoxic proteins (e.g. ricin A chain, diphtheria toxin A chain and PEA), but their application is limited due to the drawbacks shown below.

It is known that the ricin A and diphtheria toxin A chains both possess potent protein synthesis inhibitory activities, but their anticancer activities are weak since they do not smoothly transfer to ribosomes even when taken into cells via antibodies. On the other hand, PEA easily transfers to ribosomes at the target site after being taken into cells, thus exhibiting very strong cytotoxic effects, as well as strong protein synthesis inhibitory effects. It is anticipated that the protein complex of anticancer antibody and PEA will have serious adverse side effects due to receptor-mediated nonspecific toxicity, since it possesses not only antibody-mediated but also PEA receptor-mediated cytotoxicity; in addition, normal human cells mostly express a PEA receptor. In light of this situation, attempts have also been made to reduce cytotoxicity to normal human cells by modifying PEA [R. Pirker et al., Journal of Clinical Investigation, *76*, 1261 (1985); R. Pirker et al., Cancer Research, *45*, 751 (1985)], but no satisfactory results have been obtained.

Another major drawback in using anticancer antibodies as anticancer agents resides in the fact that these antibodies are for the most part mouse or rat monoclonal antibodies, i.e. proteins which are heterogenic to humans. Transfer of heterogenic protein to human bodies poses the problem of potential serious adverse side effects such as anaphylactic and other allergic reactions..

WO-A-8 800 703 relates to an immunotoxin conjugate consisting of anti-human ovarian cancer cell monoclonal antibody and Pseudomonas aeruginosa exotoxin chemically bound thereof, and its use of a cell-specific therapy of cancer.

US-A-4 545 985 relates to an immunotoxin conjugate comprising Pseudomonas aeruginosa exotoxin bound to a cancer cell line binding protein such as a monoclonal antibody against human tumor cells.

BRIEF DESCRIPTION OF THE DRAWING

Fig. 1 shows the cytotoxicity of the anti-human cancer protein complex obtained in Example 7.
Fig. 2 shows the cytotoxicity of the anti-human cancer protein complex obtained in Example 8.

DETAILED DESCRIPTION

With this technical background, the present inventors prepared and investigated Epstein-Barr virus (hereinafter also abbreviated EBV)—transformed cells which secrete anti-human cancer human monoclonal antibody or anti-PEA human monoclonal antibody, and a hybridoma of said cells and human B lymphoblastoid cells, and found that the protein complex which comprises three components, namely these two monoclonal antibodies (hereinafter also abbreviated MoAb) and PEA, acts neither on normal tissues nor normal cells, but specifically on the target cancer tissue or cells.

The present inventors also prepared a hybridoma which produces and secretes a heteroreactive, divalent hybrid antibody, a new type of antibody which reacts on cancer cells at one antigen-binding site while neutralizing and immobilizing PEA molecule at the other antigen-binding site, by cell fusion of the above-mentioned EBV-transformed cell lines which respectively produce the above two types of human MoAb or of the hybridomas of said cell lines and human B lymphoblastoid cells, and found that the protein complex which comprises said hybrid antibody and PEA can function as a very highly selective anticancer agent. The present inventors made further investigations based on these findings, developing the present invention.

Accordingly, the present invention involves an anti-human cancer protein complex which comprises (a) a ligand which is capable of binding to human cancer cells, (b) a ligand which is capable of binding to *Pseudomonas aeruginosa* exotoxin A (PEA), and (c) *Pseudomonas aeruginosa* exotoxin A (PEA) coupled to the ligand(b).

Examples of the above-mentioned anti-human cancer protein complex include 1) the anti-human cancer protein complex which comprises a protein complex obtained by covalently binding anti-human cancer human monoclonal antibody and *Pseudomonas aeruginosa* exotoxin A, and anti-*Pseudomonas aeruginosa* exotoxin A human monoclonal antibody immunologically coupled thereto, 2) the anti-human cancer protein complex which comprises a protein complex obtained by covalently binding anti-human cancer human monoclonal antibody and anti-*Pseudomonas aeruginosa* exotoxin A human monoclonal antibody, and the *Pseudomonas aeruginosa* exotoxin A immunologically coupled thereto, and 3) the anti-human cancer protein complex which comprises a heteroreactive, divalent hybrid human monoclonal antibody capable of binding to both human cancer cells, and *Pseudomonas aeruginosa* on each molecule, and *Pseudomonas aeruginosa* exotoxin A immunologically coupled thereto.

*Pseudomonas aeruginosa* exotoxin A, as referred to in the present invention, implies the toxins exemplified by the exotoxin A produced by *Pseudomonas aeruginosa* [M.L. Vasil, D. Kabat, B.H. Iglewski, Infection and Immunity, *16*, 353 (1977); G.L. Gray et al., Proceedings of National Academy of Science, USA, *81*,2645 (1984)].

For the ligand which binds to human cancer cells, any ligand can be used which is capable of specifically binding to human cancer cells. Examples of such ligands include anti-human cancer human monoclonal antibodies and their fragments capable of binding to human cancer cells (e.g. Fab, Fab′, and F-(ab′)$_2$ fragments). Anti-human cancer human monoclonal antibodies capable of binding to gastric cancer cells, lung cancer cells or epidermoid carcinoma cells are preferred; a representative example is the anti-human cancer human monoclonal antibody AGC-78, obtained in Examples given below.

For the ligand which binds to *Pseudomonas aeruginosa* exotoxin A, any ligand can be used which is capable of specifically binding to *Pseudomonas aeruginosa* exotoxin A. Examples of such ligands include anti-*Pseudomonas aeruginosa* exotoxin A human monoclonal antibodies and their fragments capable of binding to P*seudomonas aeruginosa* exotoxin A (e.g. Fab, Fab', and F(ab')$_2$ fragments, etc.). Anti-P*seudomonas aeruginosa* exotoxin A human monoclonal antibodies having capability such that 100 ng of the antibody can neutralize more than 10 ng of P*seudomonas aeruginosa* exotoxin A are preferred; representative examples are the anti-P*seudomonas aeruginosa* exotoxin human monoclonal antibodies P7E9C7 and PEA7-1-6D, obtained in Examples given below.

EBV-transformed cell lines or hybridomas which produce the above-mentioned anti-human cancer human monoclonal antibodies or anti-P*seudomonas aeruginosa* exotoxin A human monoclonal antibodies can be prepared by known methods; examples of methods which can be used include that in which antibody-producing lymphoid cells are infected with EBV to transform them into proliferative cells, followed by cloning, and that in which an EBV-transformed cell line thus obtained is further subjected to cell fusion with a human B lymphoblastoid cell line, followed by selection and cloning of the desired antibody-producing human-human hybridoma.

The antibody-producing lymphoid cells for these methods may be of any derivation, including human peripheral blood, lymph node and spleen.

Examples of the human B lymphoblastoid cell line used as the parental line include HAT (hypoxanthine-aminopterin-thymidine)-sensitive, ouabain-resistant TAW-925 (IFO 50095) [Y. Ichimori et al., Biochemical and Biophysical Research Communications, *142*, 806 (1987)].

Examples of EBV-transformed cell lines or hybridomas obtained by the above methods include the anti-human cancer human monoclonal antibody-producing human-human hybridoma AGC-78 (FERM BP-1812, IFO 50165), which reacts on gastric cancer cells (NUGC-4), lung cancer cells (LU-134) or epidermoid carcinoma cells (A431), the anti-PEA human monoclonal antibody-producing EBV-transformed cell line PEA7-1-6D (FERM BP-1809, IFO 50138), and the anti-PEA human monoclonal antibody-producing human-human hybridoma P7E9C7 (FERM BP-1810, IFO 50139), all of which can be obtained in Examples given below.

The desired human monoclonal antibodies can be produced by cultivating these cell lines or hybridomas in vitro or in vivo and subsequently purifying the produced human monoclonal antibodies by known methods.

The anti-human cancer human monoclonal antibody AGC-78 has characteristics suitable for the purpose of the present invention in that it is an antibody of the IgG type which possesses specificity and very high affinity to the above-mentioned cancer cells. Also, the anti-PEA human monoclonal antibodies PEA7-1-6D and P7E9C7 are both antibodies of the IgG type which exhibit high affinity to PEA, 100 ng of either being capable of neutralizing more than 10 ng of PEA; when incorporated in the anti-human cancer protein complex of the present invention, these antibodies completely inhibit the non-specific cytotoxicity of PEA, thus functioning efficiently in suppressing the occurrence of adverse side effects prior to reaching the target organ, tissue, or cell.

In the present invention, various known methods can be used to form protein complexes consisting of covalently bound anti-human cancer human monoclonal antibody and PEA and of covalently bound anti-human cancer human monoclonal antibody and anti-PEA human monoclonal antibody [V.P. Butler, Pharmacological Review, 29, 103 (1978); T. Kitagawa, Yuki Gosei Kagaku, 42, 283 (1984) etc.]. Examples of methods which can be used include 1) the method in which an anti-human cancer human monoclonal antibody is modified with N-succinimidyl pyridyl dithiopropionate (hereinafter also abbreviated SPDP) and reduced, after which it is added to PEA or an anti-PEA antibody, each previously modified with SPDP as well, followed by thiol exchange reaction to yield a protein complex, 2) the method in which an anti-human cancer human monoclonal antibody is modified with SPDP and reduced, after which it is added to PEA or an anti-PEA human monoclonal antibody, each previously maleimidated with N-(m-maleimidobenzoyloxy) succinimide (hereinafter also abbreviated MBS), followed by a thioether linking reaction to yield a protein complex, 3) the method in which an anti-human cancer human monoclonal antibody is pepsinized and reduced to yield Fab'-SH, followed by the addition of PEA or an anti-PEA human monoclonal antibody, each previously maleimidated or modified with SPDP, thereto to yield a protein complex, and 4) the method in which an anti-human cancer human monoclonal antibody, and PEA or an anti-PEA human monoclonal antibody are polymerized in the presence of glutaraldehyde to yield a protein complex. In addition to MBS mentioned above, N-($\gamma$-maleimidobutyryloxy) succinimide (hereinafter also abbreviated GMBS), N-($\epsilon$-maleimidocaproyloxy)-succinimide and other maleimidating agents can be used as well. In the methods mentioned above, the antibody fragments F(ab')$_2$, Fab', and Fab'' can be used in place of antibody IgG. It is also possible to form protein complexes by treating combinations of anti-human cancer human monoclonal antibody and anti-PEA human monoclonal antibody in a thoroughly reciprocal manner.

Immune complexes formed by the addition of an anti-PEA human MoAb to the protein complex of an anti-human cancer human MoAb and PEA, or of PEA to the protein complex of an anti-human cancer human MoAb and anti-PEA human MoAb, followed by reaction at 5 to 37°C for 30 minutes to 20 hours, preferably at 5 to 20°C for 2 hours, can be used as the anti-human cancer protein complex of the present invention. Since these reactions are very highly specific, it is not always necessary to use anti-PEA human MoAb or PEA with high purity, and, in addition, the desired complex can be obtained simply by mixing.

The hybridoma of the present invention, which produces a hetero-reactive, divalent hybrid human monoclonal antibody, can be obtained by fusing an anti-human cancer human MoAb-producing cell and an anti-PEA human MoAb-producing cell by a known method [G. Köhler and C. Milstein, Nature, 256, 495 (1975); J.T. Wang and R.B. Colvin, Journal of Immunology, 139, 1369 (1987)]. For example, human peripheral blood lymphocytes or cancer patient lymph node lymphocytes are transformed with EBV, after which they are fused with the HAT (hypoxanthine-aminopterin-thymidine)-sensitive, ouabain-resistant human B lymphoblastoid cell line TAW-925 by a known method; the human-human hybridoma cells thus obtained are cultivated in HAT • ouabain-containing medium (HAT • O medium), followed by selection and breeding of anti-human cancer human MoAb-producing clones. Separately, Pseudomonas aeruginosa-sensitized human peripheral blood lymphocytes are transformed with EBV into proliferative cells; anti-PEA human MoAb-producing clones are then selected and bred. To prepare a hybrid hybridoma which produces hybrid human monoclonal antibody, the above-mentioned anti-PEA human MoAb-producing EBV-transformed cell line is acclimatized, stepwise, to culture media supplemented with 8-azaguanine (hereinafter also abbreviated 8-AZG) to make it sensitive to HAT again, after which it is fused with the above-mentioned anti-PEA human MoAb-producing EBV-transformed cell line. Hybrid hybridoma selection is conducted on HAT • O medium to select and clone the hybridoma which secretes a hybrid human monoclonal antibody possessing both human cancer cell binding activities and PEA binding activities.

Methods considered applicable to the preparation of hybrid human MoAb-producing hybridoma in place of the above method include 1) the method in which an anti-human cancer human MoAb-producing EBV-transformed cell line and anti-PEA human MoAb-producing EBV-transformed cell line are fused, 2) the method in which an anti-human cancer human MoAb-producing EBV-transformed cell line and anti-PEA human MoAb producing human-human hybridoma are fused (preparation of trioma), and 3) the method in which an anti-human cancer human MoAb-producing human-human hybridoma and anti-PEA human MoAb-producing human-human hybridoma are fused (preparation of tetraoma).

The above-mentioned hybrid human monoclonal antibody can also be produced by chemically binding an anti-human cancer human monoclonal antibody and anti-PEA human monoclonal antibody fragment (e.g. Fab, Fab', F(ab')$_2$ fragments) in accordance with the method described in M.J. Glennie et. al., Journal of Immunology, *139*, 2367 (1987). Heteroreactive, divalent hybrid antibody fragments (e.g. F(ab')$_2$ fragment) capable of binding to both human cancer cells and P*seudomonas aeruginosa* exotoxin A on each molecule can be used in place of the above-mentioned hybrid human monoclonal antibody for the same purpose.

Examples of EBV-containing materials which can be used for the above procedure include the culture supernatant of the marmoset cell line B95-8 [Proceedings of National Academy of Science, USA, *70*, 190 (1973)]. The transformation can be carried out by adding an appropriate amount of the culture supernatant of the marmoset cell line B95-8 to a suspension of 0.5 to 5 X 10$^7$ cells/mℓ, preferably 1 X 10$^7$ cells/mℓ antibody-producing lymphoid cells, and carrying out infection through slight shaking at 37°C for about 1 hour, followed by cultivation for 5 to 30 days.

Cell fusion of the above-mentioned EBV-transformed cell lines, human B lymphoblastoid cell lines, antibody-producing human-human hybridomas etc. can be carried out by means of a fusogen such as Sendai virus or polyethylene glycol (hereinafter also abbreviated PEG) or electric stimulation. PEG is preferably used; its use is exemplified below, but the present invention is not limited thereto. Efficient fusion can be achieved by keeping the cells in contact with PEG with a molecular weight of 1000 to 6000, at a concentration of 10 to 80%, for 0.5 to 30 minutes, preferably PEG 6000 at 35 to 55% for 4 to 10 minutes, at 37°C.

Hybridoma selection can be achieved using HAT•O medium etc.; for this purpose, respective chemical-resistant transformants are obtained by the chemical acclimatization method using 8-AZG, 6-thioguanine (6-TG), 5-bromodeoxyuridine, or ouabain. Various selection media are used since new markers are incorporated into fused cells. Examples of such media include media supplemented with neomycin or hygromycin B [B. Sugden et al., Molecular and Cellular Biology, *5*,410(1985)].

For screening antibody-producing cells, various methods can be used. Examples of methods for determining anti-human cancer human monoclonal antibody include the mixed hemagglutination method (hereinafter also abbreviated MHA), in which the antibody is detected by the adsorption of indicator erythrocytes to tumor cells, and the cell-ELISA method, in which an antibody bound to tumor cells adhering to a microplate is detected by enzyme immunoassay (hereinafter also abbreviated ELISA). To determine anti-PEA human MoAb, ELISA using a microplate on which PEA is adsorbed as the solid phase antigen is preferred.

To determine the hybrid antibody, a system, as a modification of the cell-ELISA method, in which the antibody in the hybridoma culture supernatant sample is coupled to tumor cells adhering to a microplate, followed by the addition of enzyme-labeled PEA, may be used in place of the two above-mentioned methods.

The EBV-transformed cell line or hybridoma positive for antibody activity is immediately subjected to cloning, which can easily be carried out, usually by limiting dilution analysis. The culture supernatant of the cloned EBV-transformed cell line or hybridoma is assayed for antibody titer by any one of the above-mentioned methods, and an EBV-transformed cell line or hybridoma which stably produces an antibody with a high titer is selected, whereby the desired monoclonal EBV-transformed cell line or hybridoma can be obtained.

The above-mentioned EBV-transformed cell line or hybridoma can be cultivated as follows.

Cultivation is normally conducted in a liquid medium or in the abdominal cavity of an animal (usually a mammal, such as a nude mouse). An example of cultivation in liquid medium is described below.

Examples of the medium include basal media for animal cell cultivation [Iscove medium-Ham F12 medium 1:1 mixed medium (I • H medium), RPMI 1640 medium, etc.] as supplemented with fetal bovine serum and GIT medium (commercially available from Wako Pure Chemical Industries, Ltd., Japan) (a mammalian serum-derived composition for animal cell cultivation, produced by subjecting mammalian serum to purifying treatment including an inactivation process for contaminant microorganisms and a salting-out/desalting process; cf. Japanese unexamined patent publication No. 145088/1985).

Cultivation is normally carried out at 30 to 38°C, preferably at 37°C, for 3 to 60 days, preferably 5 to 10 days.

Purification of the antibody in the culture broth can be achieved by combining known biochemical techniques.

For example, the cell culture broth is centrifuged to separate the culture supernatant, which is then collected and subjected to salting-out (usually with ammonium sulfate or sodium sulfate). The protein precipitate thus obtained is dissolved in an appropriate solution and dialyzed, after which it is subjected to column chromatography (ion exchange column, gel filtration column, protein A column, hydroxylapatite column etc.), whereby the desired antibody can be separated and purified. For example, 1 to 30 mg of anti-human cancer human monoclonal antibody, anti-*Pseudomonas aeruginosa* exotoxin A human monoclonal antibody or hybrid human monoclonal antibody with a purity of over 80%, as calculated in protein weight ratio, can be obtained from 6 ℓ of the culture supernatant by the above procedure of separation and purification. In addition, this purified antibody preparation comprises less than 0.1% of the contents of bovine serum albumin and bovine serum globulin, both foreign proteins, and is free from the possibility of EBV contamination; these features are favourable when the preparation is administered as a therapeutic drug, for example.

The human MoAb thus obtained may be subjected to proteolytic enzyme (e.g. papain, pepsin) treatment or reducing agent treatment and thereby yielding an Fab, Fab', or F(ab')$_2$ fragment possessing binding activity to human cancer cells and/or PEA, all of which can be used for the same purpose as the human MoAb of the present invention

The anti-human cancer human MoAb produced in accordance with the present invention specifically recognizes the tumour-associated antigen on the surface of tumour cells and binds to these cells, but not to normal cells. Also, the anti-PEA human MoAb produced in accordance with the present invention specifically recognizes the antigen determinant of PEA molecule, exhibiting strong binding activity to PEA. Hybrid human MoAb possesses the antibody characteristics of the above two antibodies. These antibodies are very suitable for use as biochemical preparations, since they are homogeneous and potent.

When the anti-human cancer protein complex of the present invention contains both an anti-human cancer human monoclonal antibody or its fragment capable of binding to human cancer cells and anti-*Pseudomonas aeruginosa* exotoxin A human monoclonal antibody or its fragment capable of binding to *Pseudomonas aeruginosa* exotoxin A, it is preferable that the molar ratio be in the range of 4:1 to 1:4, more preferably 2:1 to 1:2, and ideally 1:1. It is also preferable that the latter fragment be coupled with 1 to 8 molecules, more preferably 1 to 2 molecules of *Pseudomonas aeruginosa* exotoxin A.

The anti-human cancer protein complex of the present invention can, for example, be administered per se or as injections prepared by mixing the complex with an appropriate carrier, excipient or diluent, that is pharmacologically acceptable to mammals (mice, rats, cats, dogs, swine, bovine, monkeys and humans) after germ removal by filtration through a membrane filter.

With the cytotoxic effects of PEA, the anti-human cancer protein complex of the present invention permits treatment of various cancers including gastric cancer and cancer of the lung, liver, large intestine, uterus, pancreas, kidney, gallbladder, prostate, and skin; if desired, it may be further coupled with anticancer agents other than PEA (e.g. anticancer chemotherapeutics such as methotrexate, daunomycin, vincristine, and cysplatin; cytotoxic proteins such as ricin, abrin, diphtheria toxin, and neocarzinostatin; and cytokines such as interferon and interleukin).

The dose level of the anti-human cancer protein complex of the present invention varies depending upon the type of cancer to be treated, symptoms, and route of administration; for example, when intravenously administered to lung cancer patients, the dose level is normally 0.01 to 5 mg/kg, preferably 0.03 to 0.5 mg/kg daily, as calculated as *Pseudomonas aeruginosa* exotoxin A.

The anti-human cancer protein complex thus obtained uses a ligand which binds to human cancer cells, neutralizes the adverse side effects of PEA using another ligand which binds to PEA, and functions as a therapeutic drug for cancer that is very highly selective in that it acts specifically at the target tumour site. Unlike conventional chemotherapeutics and immunotoxins, the anti-human cancer protein complex is prominently unique in that the potent cytotoxicity of PEA is completely neutralized before it reaches the target site. Furthermore, the anti-human cancer protein complex is applicable to metastatic cancer and terminal cancer as well, since it affords noticeable therapeutic effects at lower dose levels.

EXAMPLES

The present invention is described below in more detail by means of the reference examples and working examples.

Note that human-human hybridoma AGC-78, disclosed in Example 1, and hybrid hybridoma HH-1, disclosed in Example 4, have been deposited at the Institute for Fermentation, Osaka (IFO) under the respective accession numbers IFO 50165 and 50162, since March 4, 1988 and March 2, 1988; they have also been deposited at the Fermentation Research Institute (FRI), Agency of Industrial Science and Technology, Ministry of International Trade and Industry, Japan under the respective accession numbers FERM BP-1812 and FERM BP-1811, since March 23, 1988. The EBV-transformed cell line PEA7-1-6D, disclosed in Example 2, and the human-human hybridoma P7E9C7, disclosed in Example 3, have been deposited at the IFO under the respective accession numbers IFO 50138 and 50139, since August 6, 1987;

they have also been deposited at the FRI under the respective accession numbers FERM BP-1809 and FERM BP-1810, since March 23, 1988.

Reference Example 1
(Mixed hemagglutination (MHA))

① Preparation of indicator cells using human erythrocytes

In the case of IgG antibody detection, anti-D antibodies were added to 2% human erythrocytes in suspension in a phosphate buffer solution (hereinafter also abbreviated PBS), followed by incubation for 3 hr. at room temperature. The mixture was washed with PBS and again suspended in a 2% suspension. To this suspension, goat anti-human-IgG antibodies were added, followed by incubation at room temperature overnight. The mixture was then washed three times with PBS and the cells used as indicator cells.

In the case of IgM antibody detection, dried guinea pig complements were added to 2% human erythrocytes in suspension in PBS, and the cells immediately used as indicator cells.

② Target tumor cells were seeded on a Nunc Terasaki plate at 1,000 to 2,000 cells per well, followed by one day of cultivation at 37°C in a carbon dioxide incubator. After plate washing with PBS supplemented with 2% fetal calf serum (hereinafter also abbreviated FCS), the subject culture supernatant of hybridoma was added, followed by incubation for 1 to 2 hr. at room temperature. The plate was then washed with PBS supplemented with 2% FCS; a 0.2% suspension of the indicator cells prepared in ① above was then added to the plate in the amount of one drop per well. Reaction was further carried out at room temperature for 1.5 to 2 hr., when the plate was again washed with PBS supplemented with 2% FCS; hemadsorption to the tumor cells was then assessed microscopically.

Reference Example 2
(Cell-ELISA using tumor cells)

Target tumor cells were seeded on a Nunc 96-well microplate at 10,000 to 40,000 cells per well, followed by one day of cultivation at 37°C in a carbon dioxide incubator. After removing the culture supernatant, another culture supernatant of an anti-cancer antibody-producing hybridoma was added; reaction was then carried out at room temperature for 2 hr. After microplate washing with a medium supplemented with 0.2% bovine serum albumin (hereinafter also abbreviated BSA), goat anti-human-Ig antibodies labeled with horseradish peroxidase (hereinafter also abbreviated HRP) were added. Further reaction was carried out at room temperature for 2 hr. After microplate washing, a 0.1 M citrate buffer solution containing orthophenylenediamine (as the enzyme substrate) and $H_2O_2$ was added to each well; enzyme reaction was then carried out at room temperature. Following reaction termination by 1N sulfuric acid, the amount of coloring pigments was determined at a wavelength of 492 nm by means of a Multiscan (Flow Co.).

Reference Example 3
(ELISA to determine the PEA antibody (PEA-ELISA))

A 2.5 $\mu$g/m$\ell$ solution of PEA was dispensed in a 96-well microplate at 100 $\mu\ell$ per well; the microplate was then kept at 4°C for one day; PBS containing 2% casein was then added to prepare a sensitization plate. At the time of ELISA determination, the above-mentioned solution was removed and the microplate was washed with PBS; the subject culture supernatant of the EBV-transformed cell line or hybridoma was then added and reaction was carried out at room temperature for 2 hr. After microplate washing with PBS, HRP-labeled goat anti-human-Ig antibodies were added, and further reaction was carried out at room temperature for 2 hr. Enzyme reaction was then carried out by the method described in Reference Example 2, and the antibody titer was determined.

Reference Example 4
(ELISA to determine hybrid antibody (hybrid-ELISA))

The culture supernatant of hybrid antibody-producing hybridoma was added to a microplate on which tumour cells were adsorbed as described in Reference Example 2, followed by reaction for 2 hr. at room temperature. After microplate washing with a medium supplemented with 0.2% BSA, HRP-labeled PEA was added. Further reaction was carried out at room temperature for 2 hr. Enzyme reaction was then carried out by the method described in Reference Example 2, and the antibody titer was determined.

Reference Example 5
(PEA neutralizing activity test of the anti-PEA antibody)

Cells of the human B lymphoblastoid cell line BALL-1 at $5 \times 10^5$ cells/mℓ, were suspended in an I•H medium containing 10% FCS; this suspension was seeded on a 96-well microplate at 100 $\mu\ell$ per well, and the cells were used as the PEA target cells.

The culture supernatant of anti-PEA antibody-producing cells and 0.4 $\mu$g/mℓ PEA were added to the wells each at 50 $\mu\ell$ per well, followed by mixing. After cultivatin at 37°C in a carbon dioxide incubator for 5 days, the supernatant was removed at 100 $\mu\ell$ per well; 20 $\mu\ell$ of a phosphate buffer solution containing 3-(4,5-dimethylthiazol-2-yl)-diphenyltetrazolium bromide (5 mg/mℓ) was then added, followed by reaction at 37°C in a carbon dioxide incubator for 4 hr. 100 $\mu\ell$ of 10% sodium dodecylsulfate-0.01 N hydrochloric acid was then added; 24 hours later, the absorbance at a wavelength of 590 nm was determined using a Multiscan to obtain viable cell count. [MTT method; H. Tada et al., Journal of Immunological Methods, *93*, 157 (1986)].

Reference Example 6
(EBV-transformed cell line)

Lymphocytes (hereinafter also abbreviated PBL) were separated from human peripheral blood by the specific gravity centrifugation method using Ficoll-Hypaque, and suspended in an Iscove-Ham medium containing 20% FCS (I•H-20F) to a density of $1 \times 10^7$ lymphocytes/mℓ. To this suspension, an EBV-containing culture supernatant of B95-8 cells was added at a ratio by volume of 10 to 1 of the suspension; infection was carried out at 37°C for 1 hr. while the mixture was shaken gently. After infection, the lymphocytes were seeded on a 96-well microplate at $2 \times 10^4$ lymphocytes per well, followed by cultivation at 37°C in a carbon dioxide incubator for 2 to 4 weeks to yield a proliferative transformant cell line.

Reference Example 7
(Human-human hybridoma)

Cell fusion was conducted on the EBV-transformed cell line obtained in Reference Example 6 and the human B lymphoblastoid cell line TAW-925.

Cell from these cell lines were mixed together at a ratio by number of 1 to 1; the mixture was treated with 45% PEG 6000 (produced by Koch-Light Ltd.) for 7 minutes for cell fusion. After cell fusion, the fused cells were suspended in an I•H medium containing 10% FCS; this suspension was seeded on a 96-well microplate at $2 \times 10^4$ EBV-transformed cells per well, followed by cultivation at 37°C in a carbon dioxide incubator. 24 hours after initiation of the cultivation, HAT•O selection cultivation was commenced by adding an equal amount of an I•H medium containing both HAT + ouabain ($1 \times 10^{-4}$ M hypoxanthine, $4 \times 10^{-7}$ M aminopterin, $1.6 \times 10^{-5}$ M thymidine, $5 \times 10^{-6}$ M ouabain) and 10% FCS (HAT•O medium). At 3, 5, and 7 days following the day of the first addition of HAT•O medium, 100 $\mu\ell$ of the old broth was discarded and 100 $\mu\ell$ of a fresh HAT•O medium was added to continue HAT•O selection cultivation. Hybridoma proliferation occurred 10 to 14 days after cell fusion; the antibody titer of the culture supernatant was determined by one of the methods described in Reference Examples 1, 2, and 3.

Example 1
(Obtaining and cloning an anti-cancer antibody-producing hybridoma)

Human PBL was transformed by the method described in Reference Example 6; the EBV-transformed cell line thus obtained was subjected to cell fusion with the human B lymphoblasoid cell line TAW-925 by the method described in Reference Example 7, and the fused cells were subjected to HAT•O selection cultivation. After cell fusion, the fused cells were seeded on a microplate at a density of 4 to $6 \times 10^4$ cells/mℓ; the culture supernatant was collected from each of the wells in which human-human hybridoma proliferation occurred in 12 to 14 days, and subjected to MHA and cell-ELISA, respectively described in Reference Examples 1 and 2.

The hybridomas contained in the wells positive for antibody activity were subjected to cloning by limiting dilution analysis. In the cloning, BALB/c mouse thymocytes, as feeder cells, were added at $5 \times 10^5$ cells per well. About 10 to 15 days later, cell proliferation occurred; the supernatant was again subjected to

8

MHA and cell-ELISA, respectively described in Reference Examples 1 and 2. The human-human hybridoma AGC-78 (FERM BP-1812, IFO 50165), which produces an anti-cancer antibody exhibiting binding activity to gastric cancer cells (NUGC-4), lung cancer cells (LU-134) and epidermoid carcinoma cells (A 431) were thus obtained.

The anti-human-cancer human monoclonal antibody AGC-78, produced by the above hybridoma, was identified as of the human $IgG_2$ class.

The results of determination of its binding activity to various tumor cell lines, as determined by the method described in Reference Example 1 or 2, are shown in Table 1.

Table 1

| Reactivity of the Anti-Cancer Antibody AGC-78 | | |
|---|---|---|
| Target Cell | Derivation | Antibody Avidity |
| MKN-28 | Gastric cancer | - |
| MKN-45 | | - |
| NUGC-4 | | + |
| AZ-521 | | - |
| Alexander | Hepatoma | - |
| HEP-3B | | - |
| HEP-G2 | | - |
| hu-H4 | | - |
| H-1 | Bile duct cancer | - |
| $C_1$-NU | Large intestine cancer | - |
| CCK-81 | | - |
| Pc-3 | Pulmonary adenocarcinoma | - |
| Pc-9 | | - |
| Pc-14 | | - |
| OKADA | | - |
| Pc-10 | Pulmonary squamous cell carcinoma | - |
| A431 | Epidermoid carcinoma | + |
| Pc-6 | Pulmonary small cell carcinoma | - |
| N-231 | | - |
| N-417 | | - |
| LU-134 | | + |
| LU-65 | Pulmonary large cell carcinoma | - |
| K562 | Leukemia | - |
| HL-60 | | - |
| MFH-114 | Sarcoma | - |
| MFH-K8P4 | | - |
| HeLa | Cervical carcinoma | - |
| 3T3 | Others | - |

Example 2
(Obtaining and cloning an anti-PEA antibody-producing EBV-transformed cell line)

Blood serum samples collected from normal humans were assayed for anti-*Pseudomonas aeruginosa* exotoxin A antibody by ELISA; human peripheral blood lymphocytes (PBL) with a high antibody titer were then separated. The lymphocytes thus obtained were transformed with EBV in accordance with the method described in Reference Example 2; proliferative cells were bred and selected. When cell proliferation

EP 0 336 405 B1

became noticeable after 2 to 4 weeks, the culture supernatant was collected and assayed for anti-PEA antibody titer by ELISA, as described in Reference Example 3. Antibody titer was noted in 1 well, which was then subjected to cloning in the same manner as in Example 1; the transformant cell line PEA7-1-6D- (FERM BP-1809, IFO 50138), excellent in proliferativity and antibody productivity, was obtained.

The anti-PEA human monoclonal antibody PEA7-1-6D, produced by this transformant, was identified as of the human IgG$_2$ class; the neutralizing activity test described in Reference Example 5 revealed that this antibody possesses very strong neutralizing capacity such that each molecule thereof can neutralize 1 to 2 molecules of PEA.

Example 3
(Obtaining and cloning an anti-PEA antibody-producing human-human hybridoma)

Cell fusion was conducted on the anti-*Pseudomonas aeruginosa* exotoxin A human monoclonal antibody-producing EBV-transformed cell line PEA7-1-6D obtained in Example 2 and the human B lymphoblastoid cell line TAW-925 in accordance with the method described in Reference Example 7.

Hybridoma proliferation occurred about 2 weeks after cell fusion; the proliferated hybridomas were subjected to PEA-ELISA, described in Reference Example 3. Cloning was conducted on the well which showed the maximum antibody activity in the same manner as in Example 1; human-human hybridoma P7E9C7 (FERM BP-1810, IFO 50139), which stably produces anti-PEA human monoclonal antibody, was obtained.

The anti-PEA human monoclonal antibody P7E9C7, produced by this hybridoma, was identified as of the human IgG$_2$ class; the neutralizing activity test described in Reference Example 5 revealed that this antibody is an excellent neutralizing antibody in that each molecule can neutralize 1 to 2 molecules of PEA, such as the antibody PEA7-1-6D, described in Example 2.

Example 4
(Obtaining and cloning a hybrid antibody-producing hybrid hybridoma)

① Obtaining an HAT-sensitive human-human hybridoma

The anticancer antibody-producing human-human hybridoma AGC-78, obtained in Example 1, was cultivated and acclimatized in a medium supplemented with 6-thioguanine (6-TG) to make it sensitive to HAT. Cultivation was commenced with an I•H-10F medium containing 1 $\mu$M 6-TG; cultivation was continued while 6-TG concentration was increased from 1 $\mu$M in 2- to 3-fold steps. The transformants that became resistant to 100 $\mu$M 6-TG were examined for HAT sensitivity; an anticancer antibody-producing and HAT-sensitive transformant, AGC-78$^r$, was obtained.

② Cell fusion of HAT-sensitive human-human hybridoma and EBV-transformed cell line

AGC-78$^r$, mentioned in ① above, was added to the anti-PEA antibody-producing cell line PEA7-1-6D, obtained in Example 2, at a ratio of 1 to 1; cell fusion was conducted as described in Reference Example 7. The hybrid hybridoma thus obtained was subjected to selection cultivation using the HAT medium; hybrid hybridoma proliferation occurred 12 to 17 days after cell fusion.

The antibody titer of the culture supernatant was determined by the three methods of Reference Examples 1, 3, and 4; 10 wells proved positive for all three methods; these wells were then subjected to cloning by limiting dilution analysis as in Example 1; the hybrid hybridoma HH-1 (FERM BP-1811, IFO 50162) was thus obtained, which exhibited the maximum hybrid antibody activity in ELISA as described in Reference Example 4.

The hybrid human monoclonal antibody HH-1, produced by this hybrid hybridoma, was identified as of the human IgG$_2$ class. Table 2 shows the antibody titers determined by the methods described in Reference Examples 1, 3, and 4. The culture supernatant of the hybrid hybridoma HH-1, which produces the hybrid human monoclonal antibody HH-1, exhibited binding activity to both antigens of a gastric cancer cell lines NUGC-4 and PEA.

10

Table 2

| Reactivity of the Hybrid Antibody | | | |
|---|---|---|---|
| Cell Cultivation Supernatant | Antibody Titer | | |
| | MHA Test[1] | PEA-ELISA[2] | Hybrid-ELISA[3] |
| AGC-78 | $1.3 \times 10^5$ | <10 | <10 |
| PEA7-1-6D | <10 | $3.2 \times 10^3$ | <10 |
| HH-1 | $6.4 \times 10^3$ | $1.2 \times 10^3$ | $5.1 \times 10^2$ |

Note:
1) Determined in accordance with the method of Reference Example 1, using gastric cancer cell line NUGC-4.
2) Determined in accordance with the method of Reference Example 3.
3) Determined in accordance with the method of Reference Example 4, using gastric cancer cell line NUGC-4.

Example 5
(Production of monoclonal antibody)

① Obtaining an anticancer antibody and anti-PEA antibody

The human-human hybridomas AGC-78 and P7E9C7, respectively obtained in Examples 1 and 3, were each suspended in GIT medium; cultivation was continued at 37°C while cultivation volume was increased step by step. To 6 ℓ of the culture supernatant thus obtained, ammonium sulfate was added to a 47% concentration; this solution was subjected to salting-out at 4°C for 60 minutes while stirring, followed by centrifugation (10,000 rpm, 15 minutes). The precipitate thus obtained was dissolved in a 20 mM Tris buffer solution (pH 7.9) containing 50 mM NaCl; this solution was dialyzed against 1 ℓ of the same buffer solution. Two hours later, the dialyzing solution was replaced by a new one and further dialysis was conducted for 15 hr., followed by centrifugation at 10,000 rpm for 15 minutes. The supernatant was applied to a column of 10 mℓ DEAE-cellulose (Whatman DE52) equilibrated with a sufficient amount of a Tris buffer solution containing 50 mM NaCl; the fraction which passes through the column in fractionation with another Tris buffer solution containing 50 mM NaCl was concentrated. The protein fraction thus obtained was further applied to a protein A column. After thorough column washing with a phosphate buffer solution (pH 8.0) containing 50 mM NaCl, IgG antibodies were eluted with a 0.1M acetate buffer solution (pH 3.0) to yield anti-human cancer human monoclonal antibody AGC-78 and anti-PEA human monoclonal antibody P7E9C7. Each antibody thus obtained was immediately neutralized with a 0.5M carbonate buffer solution (pH 10), after which it was dialyzed against PBS and used to prepare an anti-human cancer protein complex. These antibodies were assayed for antibody purity by the SDS-polyacrylamide gel electrophoresis method of Laemli et al [Nature, *227*, 680 (1970)]; their purity was confirmed at over 95%.

② Obtaining a hybrid antibody

The hybrid hybridoma HH-1 prepared in Example 4 was cultivated in GIT medium in the same manner as in ① above; 6ℓ of the culture supernatant was subjected to salting-out with ammonium sulfate, followed by dialysis, after which it was applied to a protein A column. Elution was conducted using a 0.1 M acetate buffer solution, pH 3.0, in the same manner as in ①; the eluted IgG antibody fraction was collected and neutralized, after which it was dialyzed and concentrated using a collodion bag.

This IgG antibody fraction was then applied to a column of hydroxylapatite (0.5 × 10 cm, produced by Bio-Rad Co.) equilibrated with a 10 mM phosphate buffer solution (PB, pH 6.8) [cf. L.H. Stanker et al., Journal of Immunological Methods, *76,* 157 (1985)]. After column washing with 20 mℓ of the same 10 mM PB, linear gradient elution was conducted on a density gradient from 10 mM to 200 mM PB (40 mℓ for each density); the desired hybrid human monoclonal antibody HH-1 was then isolated.

Hybrid antibody identification was conducted by the SDS-polyacrylamide gel electrophoresis method of Laemli et al, described in ①, and the cellulose acetate membrane electrophoresis method [M.R. Suresh et al., Methods of Enzymology, *121,* 210 (1986)]; it was confirmed that this antibody possesses a purity of over 80%; it was used to prepare an anti-human cancer protein complex.

Example 6
(Preparation of anti-human cancer protein complex (1))

① Dithiopyridylation of anticancer antibody

5 mg of the anti-human cancer human MoAb AGC-78 obtained in Example 5-① was dissolved in 1 mℓ of PBS. To this solution, 50 μℓ of a 1.04 mg/mℓ SPDP-ethanol solution was added, followed by reaction at room temperature for 1 hr. The reaction mixture was passed through a column of Sephadex G-25 equilibrated with a 0.1 M phosphate buffer solution (pH 6.8) containing 5 mM EDTA to remove the excess reagent; the protein fraction thus separated was concentrated using a collodion bag.

② Maleimidation of PEA

2 mg of PEA (commercially available from Seikagaku Kogyo K.K., Japan) was dissolved in 1 mℓ of PBS. 50 μℓ of a 0.85 mg/mℓ GMBS-dimethyl-formamide solution was added to this solution, followed by reaction at room temperature for 1 hr. The reaction mixture was passed through a column of Sephadex® G-25 equilibrated with a 0.1 M phosphate buffer solution (pH 6.8) containing 5 mM EDTA to remove the excess reagent; the protein fraction thus separated was concentrated using a collodion bag.

③ Preparation of anticancer antibody-PEA protein complex

To 1 mℓ of the 2 mg/mℓ dithiopyridylated antibody obtained in ①, 150 μℓ of a 0.2 M acetate buffer solution (pH 4.5) containing 1% dithiothreitol (hereinafter also abbreviated DTT) was added; reaction was carried out at room temperature for 1 hr. After completion of the reaction, the reaction mixture was applied to a column of Sephadex® G-25 to remove the excess reagent; the protein fraction thus separated was concentrated to a volume of about 1 mℓ using a collodion bag. To this concentrate, 1 mℓ of the 2 mg/mℓ maleimidated PEA solution obtained in ② was gently added, followed by reaction at 5°C overnight. After completion of the reaction, the reaction mixture was applied to a column of PBS-equilibrated Ultrogel AcA34 to separate the anti-human cancer human MoAb-PEA protein complex from the unconjugated anti-human cancer human MoAb and PEA.

④ Immune complex of anticancer antibody-PEA protein complex and anti-PEA antibody

To 1 mℓ of the protein fraction obtained in ③, which corresponded to the anti-human cancer human MoAb-PEA protein complex, 0.5 mg of the anti-PEA human MoAb P7E9C7 obtained in Example 5-① was added; reaction was carried out at 5°C for 3 hr.; the reaction mixture was applied to a column of PBS-equilibrated Ultrogel AcA34 to separate the immune complex (anti-human cancer protein complex) from the unconjugated anti-PEA human MoAb.

Table 3 shows the cytotoxicity of the immune complex of anti-human cancer human MoAb-PEA protein complex and anti-PEA human MoAb thus obtained. It was found that the anti-human cancer human MoAb-PEA protein complex acts not only on the target cell NUGC-4 but also the non-target cell HEP-G2, while the immune complex (anti-human cancer protein complex) formed between the above protein complex and the anti-PEA human MoAb specifically exhibits cytotoxicity to the target cell NUGC-4 alone.

EP 0 336 405 B1

Table 3

| Cytotoxicity of the Immune Complex of Anticancer Antibody-PEA Protein Complex and Anti-PEA Antibody | | | |
|---|---|---|---|
| Cytotoxic Substance (6ng/ml) | Anti-PEA Antibody[1] | % Cytotoxicity | |
| | | Target Cell NUGC-4 | Nontarget Cell HEP-G2 |
| Anticancer antibody[2] PEA protein complex | - | 100 | 100 |
| | + | 60 | 0 |
| PEA | - | 98 | 82 |
| | + | 5 | 0 |

Note:

1) Neutralizing antibody P7E9C7 against PEA (100ng/ml)

2) AGC-78, an antibody reactive to gastric cancer cells (NUGC-4) and nonreactive to hepatoma cells (HEP-G2).

3) Calculated as PEA.

Example 7
(Preparation of anti-human cancer protein complex (2)

① Dithiopyridylation of anticancer antibody

A dithiopyridylated anti-human cancer human MoAb was prepared in the same manner as in Example 6-1.

② Maleimidation of anti-PEA antibody

5 mg of the anti-PEA human MoAb P7E9C7 obtained in Example 5-① was dissolved in 1 mℓ of PBS. To this solution, 50 $\mu\ell$ of a 0.93 mg/mℓ GMBS-dimethylformamide solution was added; a maleimidated anti-PEA human MoAb was prepared in the same manner as in Example 6-②.

③ Preparation of anticancer antibody-anti-PEA antibody protein complex

The dithiopyridylated anti-human cancer human MoAb was reduced and purified in the same manner as in Example 6-③; the maleimidated anti-PEA human MoAb obtained in ② was added thereto. The protein complex thus obtained was purified using a column of Ultrogel AcA34.

④ Immune complex of anticancer antibody-anti-PEA antibody protein complex and PEA

0.5 mg of PEA was added to the anti-human cancer human MoAb-anti-PEA human MoAb protein complex; the same procedure as in Example 6-④ was followed to prepare and purify the immune complex (anti-human cancer protein complex).

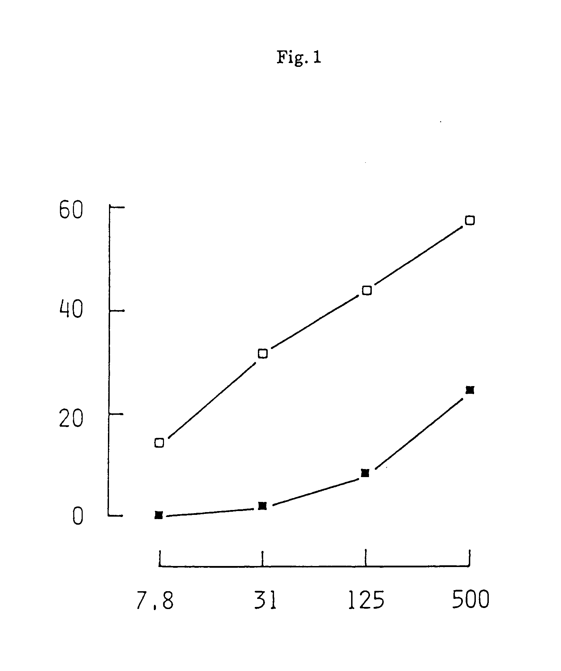

Fig. 1 shows the cytotoxicity of the immune complex of anti-human cancer human MoAb-anti-PEA human MoAb protein complex thus obtained.

In Fig. 1, □—□ shows the cytotoxicity to the target cell NUGC-4; ■—■ shows the cytotoxicity to the nontarget cell HEP-3B; the ordinate indicates % cytotoxicity and the abscissa the amount (ng/mℓ) of the immune complex (anti-human cancer protein complex).

Example 8
(Preparation of anti-human cancer protein complex (3)

① Preparation of anticancer antibody-anti-PEA antibody protein complex

1 mg of the anti-human cancer human MoAb AGC-78 obtained in Example 5-① and 1 mg of the anti-PEA human MoAb P7E9C7 were dissolved in 1 mℓ of PBS. To this solution, 50 $\mu\ell$ of a 2% aqueous

13

solution of glutaraldehyde was added; reaction was carried out at 5°C for 1 hr. Following reaction completion, the reaction mixture was applied to a column of PBS-equilibrated Ultrogel AcA34 to separate the anti-human cancer human MoAb-anti-PEA human MoAb protein complex from the unreacted antibodies.

② Immune complex of anticancer antibody-anti-PEA antibody protein complex and PEA

Using the same procedure as in Example 6-④, 0.5 mg of PEA was added to the anti-human cancer human MoAb-anti-PEA human MoAb protein complex obtained in ① above to form and purify the immune complex (anti-human cancer protein complex).

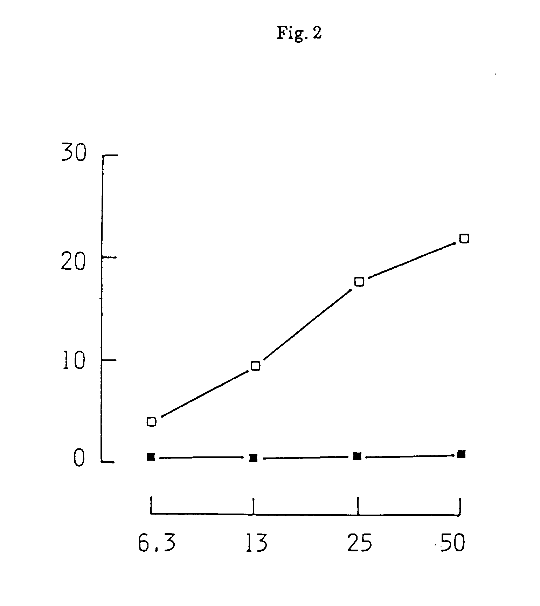

Fig. 2 shows the cytotoxicity of the immune complex of anti-human cancer human MoAb-anti-PEA human MoAb protein complex and PEA thus obtained.

In Fig. 2, □—□ shows the cytotoxocity to the target cell NUGC-4; ■—■ shows the cytotoxicity to the nontarget cell HEP-3B; the ordinate indicates % cytotoxicity and the abscissa the amount (ng/mℓ) of the immune complex (anti-human cancer protein complex).

Example 9
(Preparation of anti-human cancer protein complex (4))

0.5 mg of PEA was added to 1 mg of the hybrid human monoclonal antibody HH-1 obtained in Example 5-②; the same procedure as in Example 6-④ was followed to form and purify the immune complex (anti-human cancer protein complex).

Example 10
(Cytotoxicity of anti-human cancer protein complex ①)

A 96-well microplate seeded with tumor cells shown in Table 4 at $1 \times 10^4$ cells/100 $\mu\ell$/ well was incubated at 37°C overnight; monoclonal antibodies shown in Table 4 were then added at 0 to 50 ng/100 $\mu\ell$/well, followed by reaction at room temperature for 2 hr. After microplate washing, PEA was added to the microplate at 0 or 2.5 ng/100 $\mu\ell$/well, followed by reaction at room temperature for 2 hr. After microplate washing, a culture medium was added and cultivation was conducted at 37°C for 3 days. After completion of the cultivation, viable cells were counted by the method described in Reference Example 5. The results are shown in Table 4.

Neither AGC-78 nor P7E9C7 (parent antibodies) exhibited significant cytotoxicity to the antibody-reactive tumor cell A431 even in the presence of PEA, while the hybrid antibody HH-1 exhibited strong cytotoxicity to the target cell A 431 at 1/100 to 1/10 concentrations, relative to those of the above parent antibodies. Also, the hybrid antibody HH-1 exhibited no cytotoxicity to the nonreactive tumour cell HEP-3B even at a concentration of 500 ng/mℓ, 10 times the maximum concentration used for A431 cells.

Table 4   Potentiation of PEA Cytotoxicity by Hybrid Antibody

| Hybrid Antibody HH-1 (ng/ml) | Anticancer Antibody AGC-78 (ng/ml) | Anti-PEA Antibody P7E9C7 (ng/ml) | PEA (ng/ml) | % Cytotoxicity | |
|---|---|---|---|---|---|
| | | | | A431 Target Cells | HEP-3B Nontarget Cells |
| 0 | 0 | 0 | 0 | 0 | 0 |
| 0 | 0 | 0 | 25 | 1 | 4 |
| 0 | 0 | 500 | 0 | 4 | −[1] |
| 0 | 0 | 500 | 25 | 5 | − |
| 0 | 500 | 0 | 0 | 0 | − |
| 0 | 500 | 0 | 25 | 1 | − |
| 0 | 500 | 500 | 0 | 11 | − |
| 0 | 500 | 500 | 25 | 9 | − |
| 5 | 0 | 0 | 0 | 4 | − |
| 5 | 0 | 0 | 25 | 54 | − |
| 50 | 0 | 0 | 0 | 11 | − |
| 50 | 0 | 0 | 25 | 90 | − |
| 500 | 0 | 0 | 0 | − | 5 |
| 500 | 0 | 0 | 25 | − | 5 |

Note 1)  Not Tested

Example 11
(Cytotoxicity of anti-human cancer protein complex ②)

A 96-well microplate seeded with tumour cells shown in Table 5 at $1 \times 10^4$ cells/100μl/ well was

incubated at 37°C overnight; the anti-human cancer protein complex comprising hybrid human MoAb HH-1 and PEA, obtained in Example 9, was added, followed by cultivation at 37°C for 3 days. After completion of the cultivation, viable cells were counted by the method described in Reference Example 5. Cytotoxicity was compared between the complex and the unconjugated substance PEA. The results are shown in Table 5.

Table 5

| Cytotoxicity of Anti-Human Cancer Protein Complex | | | |
|---|---|---|---|
| Cytotoxic Substance | | % Cytotoxicity | |
| | | A431 Cells | HEP-3B Cells |
| PEA | 0.1 ng/mℓ | 0 | -[2] |
| | 0.3 | 9 | - |
| | 1 | 18 | 3 |
| | 3 | - | 18 |
| Anti-human cancer protein complex | 0.1 ng/mℓ[1] | 36 | - |
| | 0.3 | 75 | - |
| | 1 | 89 | 7 |
| | 3 | - | 11 |

Note
1) Calculated as PEA.
2) Not Tested

**Claims**

**Claims for the following Contracting States : AT, BE, CH, DE, FR, GB, IT, LI, LU, NL, SE**

1. An anti-human cancer protein complex which comprises (a) a ligand which is capable of binding to human cancer cells, (b) a ligand which is capable of binding to *Pseudomonas aeruginosa* exotoxin A, and (c) *Pseudomonas aeruginosa* exotoxin A coupled to the ligand (b).

2. The complex as claimed in claim 1, which comprises a protein complex obtained by covalently binding an anti-human cancer human monoclonal antibody and *Pseudomonas aeruginosa* exotoxin A, wherein an anti-Pseudomonas aeruginosa exotoxin A human monoclonal antibody is immunologically coupled to the protein complex.

3. The complex as claimed in claim 1, which comprises a protein complex obtained by covalently binding an anti-human cancer human monoclonal antibody and anti-*Pseudomonas aeruginosa* exotoxin A human monoclonal antibody, wherein Pseudomonas aeruginosa exotoxin A is coupled to the protein complex.

4. The complex as claimed in claim 1, which comprises a heteroreactive, divalent hybrid human monoclonal antibody capable of binding to both human cancer cells and *Pseudomonas aeruginosa* exotoxin A on each molecule and *Pseudomonas aeruginosa* exotoxin A immunologically coupled to the monoclonal antibody.

5. The complex as claimed in claim 4, in which the hybrid human monoclonal antibody is produced by a hybridoma obtained by fusing a cell which produces anti-human cancer human monoclonal antibody and another cell which produces anti-*Pseudomonas aeruginosa exotoxin* A human monoclonal antibody.

6. The complex as claimed in claim 2, 3 or 5, in which the anti-human cancer human monoclonal antibody is capable of binding to gastric cancer cells, lung cancer cells or epidermoid carcinoma cells.

7. The complex as claimed in claim 2, 3 or 5, in which the anti-Pseudomonas *aeruginosa* exotoxin A human monoclonal antibody has the capability of neutralizing *Pseudomonas aeruginosa* exotoxin A, such that 100 ng of the antibody neutralizes more than 10 ng of *Pseudomonas aeruginosa* exotoxin A.

8. The complex as claimed in claim 2, 3 or 5, in which the anti-human cancer human monoclonal antibody is produced by a cell line of an Epstein-Barr Virus-transformed cell line or a human-human hybridoma formed between an Epstein-Barr virus-transformed cell line and human B lymphoblastoid cell line.

9. The complex as claimed in claim 8 , in which the human B lymphoblastoid cell line is human B lymphoblastoid cell line TAW-925.

10. The complex as claimed in claim 2, 3 or 5, in which the anti-Pseudomonas aeruginosa exotoxin A human monoclonal antibody is produced by a cell line of an Epstein-Barr virus-transformed cell line or a human-human hybridoma formed between an Epstein-Barr virus-transformed cell line and human B lymphoblastoid cell line.

11. The complex as claimed in claim 10, in which the human B lymphoblastoid cell line is human B lymphoblastoid cell line TAW-925.

12. The complex as claimed in claim 8, in which the cell line which produces anti-human cancer human monoclonal antibody is the human-human hybridoma AGC-78.

13. The complex as claimed in claim 10,in which the cell line which produces anti-*Pseudomonas aeruginosa* exotoxin A human monoclonal antibody is the Epstein-Barr virus-transformed cell line PEA7-1-6D.

14. The complex as claimed in claim 10,in which the cell line which produces anti-*Pseudomonas aeruginosa* exotoxin A human monoclonal antibody is the human hybridoma P7E9C7.

15. The complex as claimed in claim 5, in which the hybridoma is the hybrid hybridoma HH-1, obtained by fusing the human-human hybridoma AGC-78 and the Epstein-Barr virus-transformed cell line PEA7-1-6D.

**Claims for the following Contracting States : ES, GR**

1. A process for the production of an anti-human cancer protein complex, said complex comprising (a) a ligand which is capable of bindig to human cancer cells, (b) a ligand which is capable of binding to Pseudomonas aeruginosa exotoxin A, and (c) Pseudomonas aeruginosa exotoxin A coupled to the ligand (b).

2. The process for the production of a complex as claimed in claim 1, said complex comprising a protein complex obtained by covalently binding an anti-human cancer human monoclonal antibody and Pseudomonas aeruginosa exotoxin A, wherein an anti-Pseudomonas aeruginosa exotoxin A human monoclonal antibody is immunologically coupled to the protein complex.

3. The process for the production of a complex as claimed in claim 1, said complex comprising a protein complex obtained by covalently binding an anti-human cancer human monoclonal antibody and anti-Pseudomonas aeruginosa exotoxin A human monoclonal antibody, wherein Pseudomonas aeruginosa exotoxin A is coupled to the protein complex.

4. The process for the production of a complex as claimed in claim 1, wherein a heteroreactive, divalent hybrid human monoclonal antibody capable of binding to both human cancer cells and Pseudomonas aeruginosa exotoxin A on each molecule is immunologically coupled to Pseudomonas aeruginosa exotoxin A.

5. The process as claimed in claim 4, in which the hybrid human monoclonal antibody is produced by a hybridoma obtained by fusing a cell which produces anti-human cancer human monoclonal antibody and another cell which produces anti-Pseudomonas aeruginosa exotoxin A human monoclonal antibody.

6. The process as claimed in claim 2, 3 or 5, in which the obtained anti-human cancer human monoclonal antibody is capable of binding to gastric cancer cells, lung cancer cells or epidermoid carcinoma cells.

7. The process as claimed in claim 2, 3, or 5, in which the obtained anti-Pseudomonas aeruginosa exotoxin A human monoclonal antibody has the capability of neutralizing Pseudomonas aeruginosa exotoxin A, such that 100 ng of the antibody neutralizes more than 10 ng of Pseudomonas aeruginosa exotoxin A.

8. The process as claimed in claim 2, 3, or 5, in which the anti-human cancer human monoclonal antibody is produced by a cell line of an Epstein-Barr virus-transformed cell line or a human-human hybridoma formed between an Epstein-Barr virus-transformed cell line and human B lymphoblastoid cell line.

9. The process as claimed in claim 8, in which the human B lymphoblastoid cell line is human B lymphoblastoid cell line TAW-925.

10. The process as claimed in claim 2, 3 or 5, in which the anti-Pseudomonas aeruginosa exotoxin A human monoclonal antibody is produced by a cell line of an Epstein-Barr virus-transformed cell line or a human-human hybridoma formed between an Epstein-Barr virus-transformed cell line and human B lymphoblastoid cell line.

11. The process as claimed in claim 10, in which the human B lymphoblastoid cell line is human B lymphoblastoid cell line TAW-925.

12. The process as claimed in claim 8, in which the cell line which produces anti-human cancer human monoclonal antibody is the human-human hybridoma AGC-78.

13. The process as claimed in claim 10, in which the cell line which produces anti-Pseudomonas aeruginosa exotoxin A human monoclonal antibody is the Epstein-Barr virus-transformed cell line PEA7-1-6D.

14. The process as claimed in claim 10, in which the cell line which produces anti-Pseudomonas aeruginosa exotoxin A human monoclonal antibody is the human hybridoma P7E9C7.

15. The process as claimed in claim 5, in which the hybridoma is the hybrid hybridoma HH-1, obtained by fusing the human-human hybridoma AGC-78 and the Epstein-Barr virus-transformed cell line PEA7-1-6D.

**Patentansprüche**

**Patentansprüche für folgende Vertragsstaaten : AT, BE, CH, DE, FR, GB, IT, LI, LU, NL, SE**

1. Anti-Human-Krebs-Protein-Komplex, welcher (a) einen Liganden, welcher zum Binden an menschliche Krebszellen befähigt ist, (b) einen Liganden, welcher zum Binden an Pseudomonas aeruginosa-Exotoxin A befähigt ist, und (c) an den Liganden (b) gekuppeltes Pseudomonas aeruginosa-Exotoxin A umfaßt.

2. Komplex wie in Anspruch 1 beansprucht, welcher einen Protein-Komplex umfaßt, der durch kovalentes Binden eines monoklonalen Anti-Human-Krebs-Human-Antikörpers und Pseudomonas aeruginosa-Exotoxin A erhalten wurde, worin ein monoklonaler Anti-Pseudomonas aeruginosa-Exotoxin A-Human-Antikörper immunologisch an den Protein-Komplex gekuppelt ist.

3. Komplex wie in Anspruch 1 beansprucht, welcher einen Protein-Komplex umfaßt, der durch kovalentes Binden eines monoklonalen Anti-Human-Krebs-Human-Antikörpers und monoklonalen Anti-Pseudomonas aeruginosa-Exotoxin A-Human-Antikörpers erhalten wurde, worin Pseudomonas aeruginosa-Exotoxin A an den Protein-Komplex gekuppelt ist.

4. Komplex wie in Anspruch 1 beansprucht, welcher einen heteroreaktiven, zweiwertigen, monoklonalen Hybrid-Human-Antikörper, welcher zum Binden sowohl an menschliche Krebszellen als auch an Pseudomonas aeruginosa-Exotoxin A auf jedem Molekül befähigt ist, und immunologisch an den monoklonalen Antikörper gebundenes Pseudomonas aeruginosa-Exotoxin A umfaßt.

**5.** Komplex wie in Anspruch 4 beansprucht, bei welchem der monoklonale Hybrid-Human-Antikörper durch ein Hybridom produziert wird, welches durch Fusionieren einer Zelle, welche einen monoklonalen Anti-Human-Krebs-Human-Antikörper produziert und einer weiteren Zelle, welche einen monoklonalen Anti-Pseudomonas aeruginosa-Exotoxin A-Human-Antikörper produziert, erhalten wird.

**6.** Komplex wie in Anspruch 2, 3 oder 5 beansprucht, bei welchem der monoklonale Anti-Human-Krebs-Human-Antikörper befähigt ist, an Magenkrebszellen, Lungenkrebszellen oder Epidermiskarzinomzellen zu binden.

**7.** Komplex wie in Anspruch 2, 3 oder 5 beansprucht, bei welchem der monoklonale Anti-Pseudomonas aeruginosa-Exotoxin A-Human-Antikörper befähigt ist, Pseudomonas aeruginosa-Exotoxin A zu neutralisieren, sodaß 100 ng des Antikörpers mehr als 10 ng Pseudomonas aeruginosa-Exotoxin A neutralisieren.

**8.** Komplex wie in Anspruch 2, 3 oder 5 beansprucht, bei welchem der monoklonale Anti-Human-Krebs-Human-Antikörper durch eine Zellinie einer transformierten Epstein-Barr Virus-Zellinie oder ein zwischen einer transformierten Epstein-Barr Virus-Zellinie und einer Human B-Lymphoblast-Zellinie gebildetes Human-Human-Hybridom produziert wird.

**9.** Komplex wie in Anspruch 8 beansprucht, bei welchem die Human B-Lymphoblast-Zellinie die Human B-Lymphoblast-Zellinie TAW-925 ist.

**10.** Komplex wie in Anspruch 2, 3 oder 5 beansprucht, bei welchem der monoklonale Anti-Pseudomonas aeruginosa-Exotoxin A-Human-Antikörper durch eine Zellinie einer transformierten Epstein-Barr Virus-Zellinie oder ein zwischen einer transformierten Epstein-Barr Virus-Zellinie und einer Human B-Lymphoblast-Zellinie gebildetes Human-Human-Hybridom produziert wird.

**11.** Komplex wie in Anspruch 10 beansprucht, bei welchem die Human B-Lymphoblast-Zellinie die Human B-Lymphoblast-Zellinie TAW-925 ist.

**12.** Komplex wie in Anspruch 8 beansprucht, bei welchem die Zellinie, welche den monoklonalen Anti-Human-Krebs-Human-Antikörper produziert, das Human-Human-Hybridom AGC-78 ist.

**13.** Komplex wie in Anspruch 10 beansprucht, bei welchem die Zellinie, welche den monoklonalen Anti-Pseudomonas aeruginosa-Exotoxin A-Human-Antikörper produziert, die transformierte Epstein-Barr Virus-Zellinie PEA7-1-6D ist.

**14.** Komplex wie in Anspruch 10 beansprucht, bei welchem die Zellinie, welche den monoklonalen Anti-Pseudomonas aeruginosa-Exotoxin A-Human-Antikörper produziert, das Human-Hybridom P7E9C7 ist.

**15.** Komplex wie in Anspruch 5 beansprucht, bei welchem das Hybridom das Hybrid-Hybridom HH-1 ist, welches durch Fusionieren des Human-Human-Hybridoms AGC-78 und der transformierten Epstein-Barr Virus-Zellinie PEA7-1-6D ist.

**Patentansprüche für folgende Vertragsstaaten : ES, GR**

**1.** Verfahren zur Herstellung eines Anti-Human-Krebs-Protein-Komplexes, wobei der Komplex (a) einen Liganden, welcher zum Binden an menschliche Krebszellen befähigt ist, (b) einen Liganden, welcher zum Binden an Pseudomonas aeruginosa-Exotoxin A befähigt ist, und (c) an den Liganden (b) gekuppeltes Pseudomonas aeruginosa-Exotoxin A umfaßt.

**2.** Verfahren zur Herstellung eines Komplexes wie in Anspruch 1 beansprucht, wobei der Komplex einen Protein-Komplex umfaßt, der durch kovalentes Binden eines monoklonalen Anti-Human-Krebs-Human-Antikörpers und Pseudomonas aeruginosa-Exotoxin A erhalten wurde, worin ein monoklonaler Anti-Pseudomonas aeruginosa-Exotoxin A-Human-Antikörper immunologisch an den Protein-Komplex gekuppelt ist.

**3.** Verfahren zur Herstellung eines Komplexes wie in Anspruch 1 beansprucht, wobei der Komplex einen Protein-Komplex umfaßt, der durch kovalentes Binden eines monoklonalen Anti-Human-Krebs-Human-Antikörpers und monoklonalen Anti-Pseudomonas aeruginosa-Exotoxin A-Human-Antikörpers erhalten wurde, worin Pseudomonas aeruginosa-Exotoxin A an den Protein-Komplex gekuppelt ist.

4. Verfahren zur Herstellung eines Komplexes wie in Anspruch 1 beansprucht, wobei ein heteroreaktiver, zweiwertiger, monoklonaler Hybrid-Human-Antikörper, welcher zum Binden sowohl an menschliche Krebszellen als auch an Pseudomonas aeruginosa-Exotoxin A auf jedem Molekül befähigt ist, immunologisch an Pseudomonas aeruginosa-Exotoxin A gekuppelt wird.

5. Verfahren wie in Anspruch 4 beansprucht, bei welchem der monoklonale Hybrid-Human-Antikörper durch ein Hybridom produziert wird, welches durch Fusionieren einer Zelle, welche einen monoklonalen Anti-Human-Krebs-Human-Antikörper produziert und einer weiteren Zelle, welche einen monoklonalen Anti-Pseudomonas aeruginosa-Exotoxin A-Human-Antikörper produziert, erhalten wird.

6. Verfahren wie in Anspruch 2, 3 oder 5 beansprucht, bei welchem der erhaltene monoklonale Anti-Human-Krebs-Human-Antikörper befähigt ist, an Magenkrebszellen, Lungenkrebszellen oder Epidermiskarzinomzellen zu binden.

7. Verfahren wie in Anspruch 2, 3 oder 5 beansprucht, bei welchem der erhaltene monoklonale Anti-Pseudomonas aeruginosa-Exotoxin A-Human-Antikörper befähigt ist, Pseudomonas aeruginosa-Exotoxin A zu neutralisieren, sodaß 100 ng des Antikörpers mehr als 10 ng Pseudomonas aeruginosa-Exotoxin A neutralisieren.

8. Verfahren wie in Anspruch 2, 3 oder 5 beansprucht, bei welchem der monoklonale Anti-Human-Krebs-Human-Antikörper durch eine Zellinie einer transformierten Epstein-Barr Virus-Zellinie oder ein zwischen einer transformierten Epstein-Barr Virus-Zellinie und einer Human B-Lymphoblast-Zellinie gebildetes Human-Human-Hybridom produziert wird.

9. Verfahren wie in Anspruch 8 beansprucht, bei welchem die Human B-Lymphoblast-Zellinie die Human B-Lymphoblast-Zellinie TAW-925 ist.

10. Verfahren wie in Anspruch 2, 3 oder 5 beansprucht, bei welchem der monoklonale Anti-Pseudomonas aeruginosa-Exotoxin A-Human-Antikörper durch eine Zellinie einer transformierten Epstein-Barr Virus-Zellinie oder ein zwischen einer transformierten Epstein-Barr Virus-Zellinie und einer Human B-Lymphoblast-Zellinie gebildetes Human-Human-Hybridom produziert wird.

11. Verfahren wie in Anspruch 10 beansprucht, bei welchem die Human B-Lymphoblast-Zellinie die Human B-Lymphoblast-Zellinie TAW-925 ist.

12. Verfahren wie in Anspruch 8 beansprucht, bei welchem die Zellinie, welche den monoklonalen Anti-Human-Krebs-Human-Antikörper produziert, das Human-Human-Hybridom AGC-78 ist.

13. Verfahren wie in Anspruch 10 beansprucht, bei welchem die Zellinie, welche den monoklonalen Anti-Pseudomonas aeruginosa-Exotoxin A-Human-Antikörper produziert, die transformierte Epstein-Barr Virus-Zellinie PEA7-1-6D ist.

14. Verfahren wie in Anspruch 10 beansprucht, bei welchem die Zellinie, welche den monoklonalen Anti-Pseudomonas aeruginosa-Exotoxin A-Human-Antikörper produziert, das Human-Hybridom P7E9C7 ist.

15. Verfahren wie in Anspruch 5 beansprucht, bei welchem das Hybridom das Hybrid-Hybridom HH-1 ist, welches durch Fusionieren des Human-Human-Hybridoms AGC-78 und der transformierten Epstein-Barr Virus-Zellinie PEA7-1-6D ist.

**Revendications**

**Revendications pour les Etats contractants suivants : AT, BE, CH, DE, FR, GB, IT, LI, LU, NL, SE**

1. Complexe protéinique anti-cancer humain, qui comprend (a) un ligand qui est capable de se lier à des cellules cancéreuses humaines, (b) un ligand qui est capable de se lier à l'exotoxine A de *Pseudomo-*

EP 0 336 405 B1

*nas aeruginosa*, et (c) de l'exotoxine A de *Pseudomonas aeruginosa*, accouplée au ligand (b).

2. Complexe selon la revendication 1, qui comprend un complexe protéinique obtenu par liaison covalente d'un anticorps monoclonal humain anti-cancer humain et d'exotoxine A de *Pseudomonas aeruginosa*, dans lequel un anticorps monoclonal humain anti-exotoxine A de *Pseudomonas aeruginosa* est accouplé immunologiquement au complexe protéinique.

3. Complexe selon la revendication 1, qui comprend un complexe protéinique obtenu par liaison covalente d'un anticorps monoclonal humain anti-cancer humain et d'un anticorps monoclonal humain anti-exotoxine A de *Pseudomonas aeruginosa*, dans lequel l'exotoxine A de *Pseudomonas aeruginosa* est accouplée au complexe protéinique.

4. Complexe selon la revendication 1, qui comprend un anticorps monoclonal humain hybride divalent, hétéroréactif, capable de se lier à la fois aux cellules cancéreuses humaines et à l'exotoxine A de *Pseudomonas aeruginosa* sur chaque molécule, et de l'exotoxine A de *Pseudomonas aeruginosa*, immunologiquement accouplée à l'anticorps monoclonal.

5. Complexe selon la revendication 4, dans lequel l'anticorps monoclonal humain hybride est produit par un hybridome obtenu par fusion d'une cellule qui produit l'anticorps monoclonal humain anticancer humain et d'une autre cellule qui produit l'anticorps monoclonal humain anti-exotoxine A de *Pseudomonas aeruginosa*.

6. Complexe selon la revendication 2, 3 ou 5, dans lequel l'anticorps monoclonal humain anti-cancer humain est capable de se lier à des cellules cancéreuses de l'estomac, à des cellules cancéreuses des poumons ou à des cellules de carcinome épidermoïde.

7. Complexe selon la revendication 2, 3 ou 5, dans lequel l'anticorps monoclonal humain anti-exotoxine A de *Pseudomonas aeruginosa* a une capacité de neutralisation de l'exotoxine A de *Pseudomonas aeruginosa* telle que 100 ng de l'anticorps neutralisent plus de 10 ng d'exotoxine A de *Pseudomonas aeruginosa*.

8. Complexe selon la revendication 2, 3 ou 5, dans lequel l'anticorps monoclonal humain anti-cancer humain est produit par une lignée cellulaire constituée d'une lignée cellulaire transformée par le virus d'Epstein-Barr ou d'un hybridome humain-humain formé entre une lignée cellulaire transformée par le virus d'Epstein-Barr et une lignée cellulaire de lymphoblaste B humain.

9. Complexe selon la revendication 8, dans lequel la lignée cellulaire de lymphoblaste B humain est la lignée cellulaire TAW-925 de lymphoblaste B humain.

10. Complexe selon la revendication 2, 3 ou 5, dans lequel l'anticorps monoclonal humain anti-exotoxine A de *Pseudomonas aeruginosa* est produit par une lignée cellulaire constituée d'une lignée cellulaire transformée par le virus d'Epstein-Barr ou d'un hybridome humain-humain formé entre une lignée cellulaire transformée par le virus d'Epstein-Barr et une lignée cellulaire de lymphoblaste B humain.

11. Complexe selon la revendication 10, dans lequel la lignée cellulaire de lymphoblaste B humain est la lignée cellulaire TAW-925 de lymphoblaste B humain.

12. Complexe selon la revendication 8, dans lequel la lignée cellulaire qui produit l'anticorps monoclonal humain anti-cancer humain est l' hybridome humain-humain AGC-78.

13. Complexe selon la revendication 10, dans lequel la lignée cellulaire qui produit l'anticorps monoclonal humain anti-exotoxine A de *Pseudomonas aeruginosa* est la lignée cellulaire transformée par le virus d'Epstein-Barr PEA7-1-6D.

14. Complexe selon la revendication 10, dans lequel la lignée cellulaire qui produit l'anticorps monoclonal humain anti-exotoxine A de *Pseudomonas aeruginosa* est l'hybridome humain P7E9C7.

15. Complexe selon la revendication 5, dans lequel l'hybridome est l'hybridome hybride HH-1, obtenu par

21

EP 0 336 405 B1

fusion de l'hybridome humain-humain AGC-78 et de la lignée cellulaire transformée par le virus d'Epstein-Barr PEA7-1-6D.

**Revendications pour les Etats contractants suivants : ES, GR**

1. Procédé de préparation d'un complexe protéinique anti-cancer humain, ledit complexe comprenant (a) un ligand qui est capable de se lier à des cellules cancéreuses humaines, (b) un ligand qui est capable de se lier à l'exotoxine A de *Pseudomonas aeruginosa*, et (c) de l'exotoxine A de *Pseudomonas aeruginosa*, accouplée au ligand (b).

2. Procédé de préparation d'un complexe tel que revendiqué dans la revendication 1, ledit complexe comprenant un complexe protéinique obtenu par liaison covalente d'un anticorps monoclonal humain anti-cancer humain et d'exotoxine A de *Pseudomonas aeruginosa*, dans lequel un anticorps monoclonal humain anti-exotoxine A de *Pseudomonas aeruginosa* est immunologiquement accouplé au complexe protéinique.

3. Procédé pour la préparation d'un complexe tel que revendiqué dans la revendication 1, ledit complexe comprenant un complexe protéinique obtenu par liaison covalente d'un anticorps monoclonal humain anti-cancer humain et d'un anticorps monoclonal humain anti-exotoxine A de *Pseudomonas aeruginosa*, dans lequel l'exotoxine A de *Pseudomonas aeruginosa* est accouplée au complexe protéinique.

4. Procédé de préparation d'un complexe tel que revendiqué dans la revendication 1, dans lequel un anticorps monoclonal humain hybride divalent, hétéroréactif, capable de se lier à la fois à des cellules cancéreuses humaines et à l'exotoxine A de *Pseudomonas aeruginosa* sur chaque molécule, est immunologiquement accouplé à l'exotoxine A de *Pseudomonas aeruginosa* .

5. Procédé selon la revendication 4, dans lequel l'anticorps monoclonal humain hybride est produit par un hybridome obtenu par fusion d'une cellule qui produit l'anticorps monoclonal humain anticancer humain et d'une autre cellule qui produit l'anticorps monoclonal humain anti-exotoxine A de *Pseudomonas aeruginosa*.

6. Procédé selon la revendication 2, 3 ou 5, dans lequel l'anticorps monoclonal humain anti-cancer humain obtenu est capable de se lier à des cellules cancéreuses de l'estomac, à des cellules cancéreuses des poumons ou à des cellules de carcinome épidermoïde.

7. Procédé selon la revendication 2, 3 ou 5, dans lequel l'anticorps monoclonal humain anti-exotoxine A de *Pseudomonas aeruginosa* obtenu a une capacité de neutralisation de l'exotoxine A de *Pseudomonas aeruginoso* telle que 100 ng de l'anticorps neutralisent plus de 10 ng d'exotoxine A de *Pseudomonas aeruginosa*.

8. Procédé selon la revendication 2, 3 ou 5, dans lequel l'anticorps monoclonal humain anti-cancer humain est produit par une lignée cellulaire constituée d'une lignée cellulaire transformée par le virus d'Epstein-Barr ou d'un hybridome humain-humain formé entre une lignée cellulaire transformée par le virus d'Epstein-Barr et une lignée cellulaire de lymphoblaste B humain.

9. Procédé selon la revendication 8, dans lequel la lignée cellulaire de lymphoblaste B humain est la lignée cellulaire de lymphoblaste B humain TAW-925.

10. Procédé selon la revendication 2, 3 ou 5, dans lequel l'anticorps monoclonal humain anti-exotoxine A de *Pseudomonas aeruginosa* est produit par une lignée cellulaire constituée d'une lignée cellulaire transformée par le virus d'Epstein-Barr ou d'un hybridome humain-humain formé entre une lignée cellulaire transformée par le virus d'Epstein-Barr et une lignée cellulaire de lymphoblaste B humain.

11. Procédé selon la revendication 10, dans lequel la lignée cellulaire de lymphoblaste B humain est la lignée cellulaire de lymphoblaste B humain TAW-925.

12. Procédé selon la revendication 8, dans lequel la lignée cellulaire qui produit l'anticorps monoclonal humain anti-cancer humain est l' hybridome humain-humain AGC-78.

22

**13.** Procédé selon la revendication 10, dans lequel la lignée cellulaire qui produit l'anticorps monoclonal humain anti-exotoxine A de *Pseudomonas aeruginosa* est la lignée cellulaire transformée par le virus d'Epstein-Barr PEA7-1-6D.

**14.** Procédé selon la revendication 10, dans lequel la lignée cellulaire qui produit l'anticorps monoclonal humain anti-exotoxine A de *Pseudomonas aeruginosa* est l'hybridome humain P7E9C7.

**15.** Procédé selon la revendication 5, dans lequel l'hybridome est l'hybridome hybride HH-1, obtenu par fusion de l'hybridome humain-humain AGC-78 et de la lignée cellulaire transformée par le virus d'Epstein-Barr PEA7-1-6D.

Fig. 1

Fig. 2